(19) Europäisches Patentamt
European Patent Office
Office européen des brevets

(11) **EP 3 251 590 A1**

(12) **EUROPEAN PATENT APPLICATION**
published in accordance with Art. 153(4) EPC

(43) Date of publication:
**06.12.2017 Bulletin 2017/49**

(51) Int Cl.:
**A61B 5/021** (2006.01)   **A61B 5/0402** (2006.01)
**A61B 5/024** (2006.01)

(21) Application number: **15880234.8**

(22) Date of filing: **05.06.2015**

(86) International application number:
**PCT/KR2015/005680**

(87) International publication number:
**WO 2016/122054 (04.08.2016 Gazette 2016/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **27.01.2015 KR 20150012855**

(71) Applicant: **Huinno, Co., Ltd.**
**Seongnam-si, Gyeonggi-do 463-400 (KR)**

(72) Inventor: **GIL, Yeong Joon**
**Busan 612-824 (KR)**

(74) Representative: **Glawe, Delfs, Moll**
**Partnerschaft mbB**
**von Patent- und Rechtsanwälten**
**Postfach 13 03 91**
**20103 Hamburg (DE)**

(54) **METHOD AND SYSTEM FOR MONITORING BLOOD PRESSURE IN REAL-TIME, AND NON-TRANSITORY COMPUTER-READABLE RECORDING MEDIUM**

(57)     A method of monitoring blood pressure is provided. The method comprises: calculating an estimate of deviations between every two pulse transit time (PTT) values measured at an interval of a first period, using PTT values measured for a predefined amount of time, and estimating a PTT value to be measured the first period after a current time, based on the PTT value deviation estimate and a PTT value measured at the current time; calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and calculating current systolic blood pressure and diastolic blood pressure based on an electrocardiogram (ECG) value measured at the current time, the estimated PTT value and the estimated oxygen saturation value.

FIG. 2

Printed by Jouve, 75001 PARIS (FR)

**Description**

**BACKGROUND**

1. Field of the present disclosure

The present disclosure relates to a method, system and non-transitory computer-readable recording medium for monitoring blood pressure in real time.

2. Description of the Related Art

Due to rapid developments in science and technology, the quality of life of the entire human race has been improved, bringing many changes to the medical environment. In the past, it generally takes a few hours or days to have medical images, such as X-ray, computerized tomography (CT) or functional magnetic resonance imaging (fMRI) images, interpreted.

However, as recently as ten years ago, picture archive communication systems (PACS) have been introduced which capture medical images and transmit the captured medical images to the screen of a radiologist's monitor so as for the captured medical images to be readily interpreted by the radiologist. Also, ubiquitous-Healthcare (u-Healthcare)-related medical appliances that enable patients to check their blood sugar and blood pressure anytime anywhere have been widely distributed and are increasingly being used at homes or offices by many diabetic or hypertensive patients.

In particular, in the case of hypertension, which is known to be a major cause of various diseases and has an ever-increasing prevalence, systems are needed for regularly measuring blood pressure and reporting it in real time, and research has been conducted on such systems.

Conventionally, a u-Healthcare technique has been suggested in which a blood pressure measuring sensor is inserted into the pulmonary artery of a patient with a chronic heart disease to measure the blood pressure of the patient and transmit the result of the measurement to a doctor, in real time, via radio communication and the doctor delivers a prescription to the patient while monitoring variations in the pulmonary blood pressure of the patient from a remote place. This u-Healthcare technique has advantages in that the number of the patient's visits to the doctor can be considerably reduced. However, the conventional u-Healthcare technique involves a rather invasive measurement process, even though it can continuously and precisely measure blood pressure, and may thus face many difficulties and risks (such as possible damage or inflammation to the arteries).

Accordingly, research has been carried out not only on systems capable of non-invasively measuring blood pressure in real time without the need to insert a blood pressure measuring sensor in the artery, but also on ways to allow a user to correct his or her blood pressure through biofeedback of the blood pressure monitored and measured in a ubiquitous environment.

In the meantime, there has been introduced a blood pressure measurement method in which a cuff is attached onto the arm of a user to measure the blood pressure. However, this blood pressure measurement method requires either the user or somebody else (in a case when the user is busy working or resting) to operate a blood pressure measuring device to measure blood pressure. Accordingly, it may be difficult to continuously measure blood pressure. Also, it takes more than dozens of seconds to measure blood pressure.

Particularly, in order to alert people to the danger of hypertension and thus to help them receive timely care in case of an emergency, a technique is needed to continuously measure blood pressure and report it in real time so as to help people prevent and manage hypertension.

**SUMMARY**

The present disclosure has been made to address at least the problems and disadvantages described above, and to provide at least the advantages described below.

An embodiment of the present disclosure provides a method and system for calculating blood pressure without a time delay by blood pressure without a time delay by calculating an estimate of deviations between every two pulse transit time (PTT) values measured at an interval of a first period, using PTT values measured for a predefined amount of time, estimating a PTT value to be measured the first period after a current time, based on the PTT value deviation estimate and a PTT value measured at the current time, calculating an estimate of deviations between every two oxygen saturation ($SpO_2$) values measured at an interval of a second period, using oxygen saturation values measured for the predefined amount of time, estimating an oxygen saturation value to be measured the second period after the current time, based on the oxygen saturation value deviation estimate and an oxygen saturation value measured at the current time, and calculating current systolic or diastolic blood pressure based on an electrocardiogram (ECG) value measured at the current time, the estimated PTT value and the estimated oxygen saturation value.

However, the present disclosure is not restricted to those set forth herein. The above and other embodiments of the present disclosure will become more apparent to one of ordinary skill in the art to which the present disclosure pertains by referencing the detailed description of the present disclosure given below.

In accordance with an embodiment of the present disclosure, a method of monitoring blood pressure, includes: calculating an estimate of deviations between every two PTT values measured at an interval of a first period, using PTT values measured for a predefined amount of time, and estimating a PTT value to be measured the first period after a current time, based on the PTT value deviation estimate and a PTT value measured at the current time; calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and calculating current systolic blood pressure based on an ECG value measured at the current time, the estimated PTT value and the estimated oxygen saturation value.

In accordance with another embodiment of the present disclosure, a method of monitoring blood pressure, includes: calculating an estimate of deviations between every PTT values measured at an interval of a first period, using PTT values measured for a predefined amount of time, and estimating a PTT value to be measured the first period after a current time, based on the PTT value deviation estimate and a PTT value measured at the current time; calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and calculating current diastolic blood pressure based on the estimated PTT value and the estimated oxygen saturation value.

In accordance with another embodiment of the present disclosure, a system for monitoring blood pressure, includes: a deviation estimation unit calculating an estimate of deviations between every two PTT values measured at an interval of a first period, using PTT values measured for a predefined amount of time, estimating a PTT value to be measured the first period after a current time, based on the PTT value deviation estimate and a PTT value measured at the current time, calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and a blood pressure calculation unit calculating current systolic blood pressure based on an ECG value measured at the current time, the estimated PTT value and the estimated oxygen saturation value.

In accordance with another embodiment of the present disclosure, a system for monitoring blood pressure, includes: a deviation estimation unit calculating an estimate of deviations between every PTT values measured at an interval of a first period, using PTT values measured for a predefined amount of time, estimating a PTT value to be measured the first period after a current time, based on the PTT value deviation estimate and a PTT value measured at the current time, calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and a blood pressure calculation unit calculating current diastolic blood pressure based on the estimated PTT value and the estimated oxygen saturation value.

In accordance with another embodiment of the present disclosure, a non-transitory computer-readable recording medium is provided having recorded thereon, a computer program for executing any one of the aforementioned methods of monitoring blood pressure.

According to the present disclosure, biometric signals such as ECG, photoplethysmogram (PPG), and oxygen saturation signals are measured only for a predefined amount of time only at an initial use. Once the biometric signals are measured, it is possible to continue to precisely calculate blood pressure without a time delay.

Other features and embodiments will be apparent from the following detailed description, the drawings, and the claims.

## BRIEF DESCRIPTION OF THE DRAWINGS

FIG. 1 is a schematic block diagram illustrating an entire system according to an embodiment of the present disclosure.
FIG. 2 is a block diagram illustrating an apparatus for monitoring blood pressure, according to an embodiment of the present disclosure.

FIGS. 3 and 4 are graphs showing pulse transit time (PTT) values measured for two minutes according to an embodiment of the present disclosure and deviations among the PTT values.

**DETAILED DESCRIPTION OF THE EMBODIMENTS**

Embodiments of the present disclosure are described in detail below with reference to the accompanying drawings. The embodiments of the present disclosure are sufficiently described in detail such that those skilled in the art may carry out the present disclosure. It should be understood that although various embodiments of the present disclosure are different from each other, they need not be mutually exclusive. For example, in regard to an embodiment, specific forms, structures, and characteristics described herein can be realized through another embodiment without departing from the spirit and scope of the present disclosure. Moreover, it should be understood that locations or arrangements of separate elements within the disclosed embodiments can be changed without departing from the spirit and scope of the present disclosure. Accordingly, detailed descriptions which will be given below are not intended to be restrictive, and the scope of the present disclosure, if properly described, should be limited only by the accompanying claims and equivalents thereof. Similar reference numerals shown in the drawings denote elements performing identical or similar functions in several aspects.

Embodiments will hereinafter be described with reference to the accompanying drawings.

Structure of Entire System

A system for monitoring blood pressure, according to an embodiment of the present disclosure will hereinafter be described.

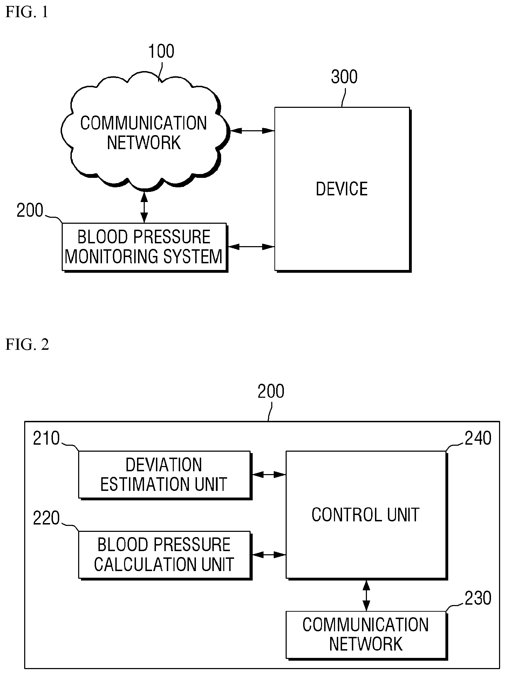

FIG. 1 is a schematic block diagram illustrating an entire system according to an embodiment of the present disclosure.

Referring to FIG. 1, the system may include a communication network 100, a system 200 (hereinafter, the blood pressure monitoring system 200) for monitoring blood pressure, and a device 300.

The communication network 100 may not necessarily be limited to a particular communication method such as a wired or wireless communication method, and may be implemented as various communication networks such as a local area network (LAN), a metropolitan area network (MAN) or a wide area network (WAN). Examples of the communication network 100 may include LANs such as a Wireless-Fidelity (Wi-Fi) network, a Wi-Fi Direct network, a Long-Term Evolution (LTE) Direct network, and a Bluetooth network that are already well known, but the present disclosure is not limited thereto. That is, the communication network 100 may at least partially include a typical wired/wireless communication network, a typical telephone network or a typical wired/wireless television communication network.

The blood pressure monitoring system 200 calculates blood pressure without a time delay by calculating an estimate of deviations between every two pulse transit time (PTT) values measured at an interval of a first period, using PTT values measured for a predefined amount of time, estimating a PTT value to be measured the first period after a current time, based on the PTT value deviation estimate and a PTT value measured at the current time, calculating an estimate of deviations between every two oxygen saturation values measured at an interval of a second period, using oxygen saturation values measured for the predefined amount of time, estimating an oxygen saturation value to be measured the second period after the current time, based on the oxygen saturation value deviation estimate and an oxygen saturation value measured at the current time, and calculating current systolic or diastolic blood pressure based on an electrocardiogram (ECG) value measured at the current time, the estimated PTT value and the estimated oxygen saturation value.

The functions of the blood pressure monitoring system 200 will be described later in further detail. The blood pressure monitoring system 200 has been described above, but the present disclosure is not limited thereto. That is, some of the functions or elements of the blood pressure monitoring system 200 may be implemented or incorporated into the device 300, if necessary.

The device 300 is a digital device capable of accessing and communicating with the blood pressure monitoring system 200, and any digital device equipped with memory means and a microprocessor and having computational capabilities may be used as the device 300. The device 300 may be a wearable device such as smart glasses, a smart watch, a smart band, a smart ring, or a smart necklace, or a rather traditional device such as a smartphone, a smart pad, a desktop computer, a notebook computer, a workstation, a personal digital assistant (PDA), a web pad, or a mobile phone. The device 300 may measure a biometric signal for monitoring a blood pressure from the human body or may provide blood pressure monitoring information to a user.

The device 300 may also include application programs for performing predetermined functions. The application programs may exist in the device 300 in the form of program modules. The program modules may be similar to a deviation estimation unit 210, a blood pressure calculation unit 220, a communication unit 230 and a control unit 240 of the blood pressure monitoring system 200. The application programs may be replaced with hardware or firmware devices capable of performing the same functions as, or at least equivalent functions to, the application programs, if necessary.

Structure of Blood Pressure Monitoring System

The structure and functions of the blood pressure monitoring system 200, which plays an important role in realizing the present disclosure, will hereinafter be described.

FIG. 2 is a block diagram illustrating an apparatus for monitoring blood pressure, according to an embodiment of the present disclosure.

Referring to FIG. 2, the blood pressure monitoring system 200 may include a deviation estimation unit 210, a blood pressure calculation unit 220, a communication unit 230, and a control unit 240. At least some of the deviation estimation unit 210, the blood pressure calculation unit 220, the communication unit 230, and the control unit 240 may be program modules communicating with an external system (not illustrated). The program modules may be included in the blood pressure monitoring system 200 in the form of operating systems, application program modules or other program modules, and may be physically stored in a memory device that is already well known. The program modules may also be stored in a remote memory device capable of communicating with the blood pressure monitoring system 200. The program modules may include routines, sub-routines, programs, objects, components, and data structures that perform particular tasks or particular abstract data types, but the present disclosure is not limited thereto.

The deviation estimation unit 210 may calculate an estimate of deviations between every two PTT values measured at an interval of a first period, using PTT values measured for a predefined amount of time, and may estimate a PTT value to be measured the first period after a current time, based on the PTT value deviation estimate and a PTT value measured at the current time.

Also, the deviation estimation unit 210 may calculate an estimate of deviations between every two oxygen saturation values measured at an interval of a second period, using oxygen saturation values measured for the predefined amount of time, and may estimate an oxygen saturation value to be measured the second period after the current time, based on the oxygen saturation value deviation estimate and an oxygen saturation value measured at the current time.

More specifically, the predefined amount of time, which is a period for securing sufficient PTT and oxygen saturation data at an initial use, may be set to be longer than the first period or the second period. For example, in response to first and second periods for calculating systolic blood pressure being 8 seconds and 42 seconds, respectively, the predefined amount of time for measuring PTT values and oxygen saturation values at an initial use may be set to be longer than 42 seconds.

The PTT value to be measured the first period after the current time may be estimated by adding the PTT value deviation estimate to the PTT value measured at the current time. Similarly, the oxygen saturation value to be measured the second period after the current time may be estimated by adding the oxygen saturation value deviation estimate to the oxygen saturation value measured at the current time.

The PTT value deviation estimate may be an average of the deviations between every two PTT values measured at the interval of the first period. Similarly, the oxygen saturation value deviation estimate may be an average of the deviations between every two oxygen saturation values measured at the interval of the second period.

The deviation estimation unit 210 may calculate the PTT value deviation estimate or the oxygen saturation value deviation estimate based on all the deviations between every two PTT values measured at the interval of the first period or all the deviations between every two oxygen saturation values measured at the interval of the second period except for outliers. For example, the deviation estimation unit 210 may calculate the PTT value deviation estimate based on all the deviations between every two PTT values measured at the interval of the first period, except for those that fall in the top 10% range and those that fall in the bottom 10% range, i.e., based on the PTT value deviations that fall in the middle 80% range. For this, the deviation estimation unit 210 may arrange the PTT value deviations in a predetermined order, for example, in ascending or descending order.

The blood pressure estimation unit 220 may calculate the current systolic blood pressure based on an ECG value measured at the current time, the estimated PTT value and the estimated oxygen saturation value. The blood pressure estimation unit 220 may calculate the current diastolic blood pressure based on the estimated PTT value and the estimated oxygen saturation value.

The calculation of systolic or diastolic blood pressure will hereinafter be described.

Systolic blood pressure and diastolic blood pressure may be calculated based on an ECG value, a PTT value and an oxygen saturation value, as indicated by Equations (1) and (2):

$$\mathrm{SBP}(t) = (a \times \mathrm{ECG}_{(t+24s)}) + (b \times 1/(\mathrm{PTT}_{(t+32s)})) + (c \times 1/(\mathrm{SpO}_{2(t+76s)})) + d \quad \ldots(1);$$

and

$$DBP(t) = (e \times 1/(PTT_{(t+70s)})) + (f \times 1/(SpO_{2(t+100s)})) + g \quad …(2).$$

Equations (1) and (2) may be simplified through time shift into Equations (3) and (4):

$$SBP(t') = (a \times ECG_{(t')}) + (b \times 1/(PTT_{(t'+8s)})) + (c \times 1/(SpO_{2(t'+42s)})) + d \quad …(3);$$

and

$$DBP(t') = (e \times 1/(PTT_{(t'+46s)})) + (f \times 1/(SpO_{2(t'+76s)})) + g \quad …(4).$$

In Equations (1) through (4), SBP(t) and DBP(t) denote systolic blood pressure and current diastolic blood pressure, respectively, ECG(t), PTT(t) and $SpO_2$(t) denote an ECG value, a PTT value and an oxygen saturation value, respectively, a, b, c, d, e, f and g denote constants (that may vary depending on the environment in which, or the target from which, blood pressure is measured). For example, the constants a, b, c, d, e, f, and g may be 0.115, 4.132, 509.819, - 454.111, -3.015, -304.556, and 410.62, respectively.

Referring to Equation (3), to calculate current systolic blood pressure, not only an ECG value $ECG_{(t')}$ measured at the current time, but also a PTT value $PTT_{(t'+8s)}$ measured 8 seconds after the current time and an oxygen saturation value $SpO_{2(t'+42s)}$ measured 42 seconds after the current time, are needed, and thus, it inevitably takes at least 42 seconds to calculate the current systolic blood pressure. Similarly, referring to Equation (4), to calculate current diastolic blood pressure, a PTT value $PTT_{(t'+46s)}$ measured 46 seconds after the current time and an oxygen saturation value $SpO_{2(t'+76s)}$ measured 76 seconds after the current time, are needed, and thus, it inevitably takes at least 76 seconds to calculate the current diastolic blood pressure.

On the other hand, according to the present embodiment, a PTT value and an oxygen saturation value to be measured a predetermined amount of time after the current time can be estimated, as discussed above with regard to the deviation estimation unit 210 and the blood pressure calculation unit 220. Accordingly, the current systolic blood pressure and the current diastolic blood pressure can be calculated without a time delay of several seconds.

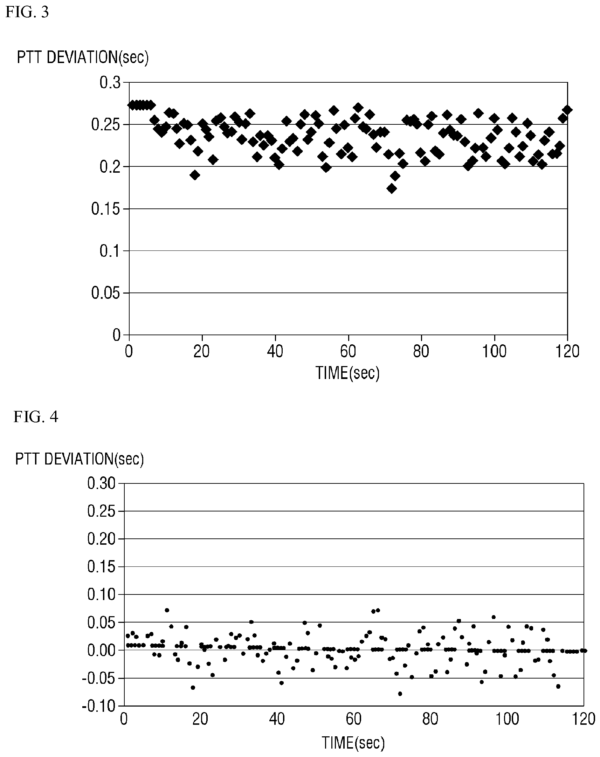

FIGS. 3 and 4 are graphs showing PTT values measured for two minutes according to an embodiment of the present disclosure and deviations among the PTT values.

More specifically, FIG. 3 show PTT values measured from a first test subject for two minutes (or 120 seconds). Referring to FIG. 3, the PTT values measured from the first test subject generally range from 200 msec to 300 msec.

FIG. 4 show deviations between every two PTT values (i.e., $PTT_{(t')}$ - $PTT_{(t'+8)}$) measured 8 seconds apart. Referring to FIG. 4, the average of the PTT value deviations may be almost zero, and particularly, about 0.00214 seconds.

Although not specifically illustrated, deviations between every two oxygen saturation values measured at an interval of a predetermined amount of time may be estimated using almost the same method as that of FIGS. 3 and 4. More specifically, test results show that the average of deviations (i.e., $SpO_{2(t')}$ - $SpO_{2(t'+8)}$) between every two oxygen saturation values measured 42 seconds apart is 0.17%.

Equation (3), which is for calculating systolic blood pressure using biometric signals that are actually measured, may be transformed into Equation (5), which is for calculating systolic blood pressure using biometric signals measured based on average deviations, and Equation (5) is as follows:

$$SBP(t') = (a \times ECG_{(t')}) + (b \times 1/(PTT_{(t')} - 0.00214)) + (c \times 1/(SpO_{2(t')} - 0.0017)) + d \quad …(5).$$

In the present embodiment, test results show that systolic blood pressure calculated by Equation (3), which uses biometric signals that are actually measured with time delays, is 127.1053 mmHg, and that systolic blood pressure calculated by Equation (5), which uses biometric signals that are estimated based on average deviations, is 125.7324 mmHg. That is, the systolic blood pressure calculated by Equation (5), which uses estimated biometric signals according to the present embodiment, and the systolic blood pressure calculated by Equation (3), which uses measured biometric signals, is only about 1%. Even though not specifically mentioned herein, tests were conducted on fifty other test subjects than the first test subject, and the results confirm that the error rate between systolic blood pressure calculated on estimated biometric signals according to the present embodiment and systolic blood pressure calculated based on measured biometric signals is an average of about 1%.

According to the present embodiment, current systolic blood pressure can be precisely calculated without a time delay.

The calculation of systolic blood pressure has been described above, but it is obvious that the present disclosure can also be directly applied to the calculation of diastolic blood pressure. The communication unit 230 may allow the blood pressure monitoring system 200 to communicate with an external device.

The control unit 240 controls the flow of data among the deviation estimation unit 210, the blood pressure calculation unit 220 and the communication unit 230. That is, the control unit 240 may control the flow of data from an external device or the flow of data between the elements of the blood pressure monitoring system 200, and may thus control the deviation estimation unit 210, the blood pressure calculation unit 220 and the communication unit 230 to perform their respective functions.

Embodiments of the present disclosure may be implemented in the form of program instructions which can be performed by various computing devices to be thereby recorded in a non-transitory computer-readable recording medium. The non-transitory computer-readable recording medium may include program instructions, data files, and data structures alone or a combination thereof. The program instructions recorded in the recording medium may be specially designed and configured for the present disclosure, or be something well-known to those skilled in the field of computer software. The non-transitory computer-readable recording medium may include magnetic media such as hard disks, floppy disks and magnetic tapes, optical media such as a Compact Disc Read-Only Memory (CD-ROM) and a Digital Versatile Disc (DVD), magneto-optical media such as floptical disks, and hardware devices such as a Read-Only Memory (ROM), a Random Access Memory (RAM) and a flash memory, which are specially configured to store and perform program instructions. Further, the program instructions include a machine language code generated by a compiler and a high-level language code executable by a computer through an interpreter and the like. The hardware devices may be configured to operate as one or more software modules to perform the operations of the present disclosure, and vice versa.

As described above, although the present disclosure has described specific matters such as concrete components, the embodiments and the drawings are provided merely to assist in a general understanding of the present disclosure, and the present disclosure is not limited to the embodiments. Various modifications and changes can be made from the description by those skilled in the art.

Accordingly, the spirit and scope of the present disclosure should not be limited or determined by the above-described embodiments, and it should be noted that not only the claims which will be described below but also their equivalents fall within the spirit and scope of the present disclosure.

## Claims

1. A method of monitoring blood pressure, comprising:

   calculating an estimate of deviations between every two pulse transit time (PTT) values measured at an interval of a first period, using PTT values measured for a predefined amount of time, and estimating a PTT value to be measured the first period after a current time, based on the PTT value deviation estimate and a PTT value measured at the current time;
   calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and
   calculating current systolic blood pressure based on an electrocardiogram (ECG) value measured at the current time, the estimated PTT value and the estimated oxygen saturation value.

2. The method of claim 1, wherein the predefined amount of time is set to be longer than whichever of the first and second periods is longer than the other.

3. The method of claim 1, wherein the PTT value to be measured the first period after the current time is estimated by adding the PTT value deviation estimate to the PTT value measured at the current time and the oxygen saturation value to be measured the second period after the current time is estimated by adding the oxygen saturation value deviation estimate to the oxygen saturation value measured at the current time.

4. The method of claim 1, wherein the PTT value deviation estimate is an average of the deviations between the every two PTT values measured at the interval of the first period and the oxygen saturation value deviation estimate is an average of the deviations between the every two oxygen saturation values measured at the interval of the second period.

5. The method of claim 1, wherein the PTT value deviation estimate is calculated based on all the deviations between the every two PTT values measured at the interval of the first period, except for outliers that fall outside a predefined range, and the oxygen saturation value deviation estimate is calculated based on all the deviations between the every two oxygen saturation values measured at the interval of the second period, except for outliers that fall outside a predefined range.

6. The method of claim 1, wherein the first period is set to be shorter than the second period.

7. The method of claim 1, wherein the calculating the current systolic blood pressure, comprises calculating the current systolic blood pressure in real time without waiting for PTT values and oxygen saturation values to be measured for the predefined amount of time.

8. A method of monitoring blood pressure, comprising:

calculating an estimate of deviations between every two PTT values measured at an interval of a first period, using PTT values measured for a predefined amount of time, and estimating a PTT value to be measured the first period after a current time, based on the PTT value deviation estimate and a PTT value measured at the current time;
calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and
calculating current diastolic blood pressure based on the estimated PTT value and the estimated oxygen saturation value.

9. A system for monitoring blood pressure, comprising:

a deviation estimation unit calculating an estimate of deviations between every two PTT values measured at an interval of a first period, using PTT values measured for a predefined amount of time, estimating a PTT value to be measured the first period after a current time, based on the PTT value deviation estimate and a PTT value measured at the current time, calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and
a blood pressure calculation unit calculating current systolic blood pressure based on an ECG value measured at the current time, the estimated PTT value and the estimated oxygen saturation value.

10. A system for monitoring blood pressure, comprising:

a deviation estimation unit calculating an estimate of deviations between every PTT values measured at an interval of a first period, using PTT values measured for a predefined amount of time, estimating a PTT value to be measured the first period after a current time, based on the PTT value deviation estimate and a PTT value measured at the current time, calculating an estimate of deviations between every two oxygen saturation values measured at an interval of a second period, using oxygen saturation values measured for the predefined amount of time, and estimating an oxygen saturation value to be measured the second period after the current time, based on the oxygen saturation value deviation estimate and an oxygen saturation value measured at the current time; and
a blood pressure calculation unit calculating current diastolic blood pressure based on the estimated PTT value and the estimated oxygen saturation value.

FIG. 1

FIG. 2

FIG. 3

PTT DEVIATION(sec)

FIG. 4

PTT DEVIATION(sec)

## INTERNATIONAL SEARCH REPORT

| International application No. |
|---|
| **PCT/KR2015/005680** |

### A. CLASSIFICATION OF SUBJECT MATTER

*A61B 5/021(2006.01)i, A61B 5/0402(2006.01)i, A61B 5/024(2006.01)i*

According to International Patent Classification (IPC) or to both national classification and IPC

### B. FIELDS SEARCHED

Minimum documentation searched (classification system followed by classification symbols)
A61B 5/021; A61B 5/024; A61B 5/02; A61B 5/022; A61B 5/0402

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: pulse wave, oxygen saturation, electrocardiogram

### C. DOCUMENTS CONSIDERED TO BE RELEVANT

| Category* | Citation of document, with indication, where appropriate, of the relevant passages | Relevant to claim No. |
|---|---|---|
| Y | KR 10-2008-0017525 A (IUCF-HYU (INDUSTRY-UNIVERSITY COOPERATION FOUNDATION HANYANG UNIVERSITY)) 27 February 2008 See abstract, figures 1-4, claims 1-16. | 1-2,6-10 |
| A | | 3-5 |
| Y | JP 06-319706A (FUKUDA DENSHI CO., LTD.) 22 November 1994 See abstract, figures 1-6, claims 1-4. | 1-2,6-10 |
| A | | 3-5 |
| Y | JP 11-318838A (NIPPON COLIN CO., LTD.) 24 November 1999 See abstract, figures 1-11, claim 1, paragraphs [0061]-[0068]. | 1-2,6-10 |
| A | KR 10-1308522 B1 (PUSAN NATIONAL UNIVERSITY INDUSTRY-UNIVERSITY COOPERATION FOUNDATION) 17 September 2013 See abstract, figures 1-5, claims 1-6. | 1-10 |

☐ Further documents are listed in the continuation of Box C.　☒ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>24 SEPTEMBER 2015 (24.09.2015) | Date of mailing of the international search report<br><br>**24 SEPTEMBER 2015 (24.09.2015)** |
| Name and mailing address of the ISA/KR<br>Korean Intellectual Property Office<br>Government Complex-Daejeon, 189 Seonsa-ro, Daejeon 302-701,<br>Republic of Korea<br>Facsimile No. 82-42-472-7140 | Authorized officer<br><br><br>Telephone No. |

Form PCT/ISA/210 (second sheet) (January 2015)

# EP 3 251 590 A1

**INTERNATIONAL SEARCH REPORT**
Information on patent family members

International application No.

**PCT/KR2015/005680**

| Patent document cited in search report | Publication date | Patent family member | Publication date |
|---|---|---|---|
| KR 10-2008-0017525 A | 27/02/2008 | NONE | |
| JP 06-319706A | 22/11/1994 | NONE | |
| JP 11-318838A | 24/11/1999 | EP 0956815 A1 | 17/11/1999 |
| | | JP 03330079 B2 | 30/09/2002 |
| | | JP 11318838 A | 24/11/1999 |
| | | US 06036651 A | 14/03/2000 |
| KR 10-1308522 B1 | 17/09/2013 | NONE | |

Form PCT/ISA/210 (patent family annex) (January 2015)

12